# EUROPEAN PATENT APPLICATION

(11) **EP 3 868 855 A1**
(43) Date of publication of application: **25.08.2021**
(21) Application number: 21160672.8
(22) Date of filing: 22.06.2007
(51) Int. Cl.: C12C 5/00, C12H 1/22, C12C 11/00, C12N 9/62

(54) **IMPROVED BREWING PROCESS**

(30) Priority: 13.07.2006 EP 06117178; 27.02.2007 US 903539 P; 04.05.2007 US 924236 P
(62) Divisional of application: 11181073.5
(71) Applicant: DSM IP Assets B.V., 6411 TE Heerlen (NL)
(72) Inventor: NGUYEN, Minh-Tam, 6100 AA ECHT (NL); EDENS, Luppo, 6100 AA ECHT (NL); VAN ROON, Jeroen Louis, 6100 AA ECHT (NL)
(74) Representative: DSM Intellectual Property

(57) **Abstract**

The present invention relates to the use of a proline-specific protease for accelerating the beer brewing process. In particular, it relates to the use of a proline-specific protease for accelerating the beer brewing process by shortening and simplifying the stabilisation phase of beer production. The stabilisation period as such can be omitted from the beer making process hereby saving significant costs and adding to the flexibility of beer producing plants.

## Description

### Field of the invention

The present invention relates to a method for beer brewing. In particular, it relates to an accelerated method for brewing beer involving a proline-specific protease.

### Background of the invention

In the traditional beer making process, wort obtained by extracting malt is inoculated with beer yeast and fermentation is allowed to proceed for about 7 days with a maximum temperature between 8-15°C depending on whether a cold or warm fermentation process is used. This fermentation is also known as primary fermentation. Once fermentation is complete and most of the fermentable sugars have been converted to alcohol, the yeasts settle out. In the case of lager beer, the yeasts collect at the bottom of the fermentation, in other beers they collect at the top of the fermentation. The "green" beer resulting from this primary fermentation still contains some non-settled yeasts as well as relatively high levels of undesirable flavor components, notably diketones, such as diacetyl and acetyl aldehyde.

The conversion of these latter undesirable flavor components into bland tasting compounds is an important aspect of the subsequent beer maturation phase. Especially the reduction of diacetyl, a compound with a buttery off-flavor, into tasteless acetoin is a time consuming process but of paramount importance. In the conventional beer process this maturation stage takes place for about a week. In the subsequent phase of beer brewing, the so-called stabilisation phase, the beer is retained to promote formation of protein polyphenol aggregates, followed by removal of precipitated aggregates. These protein-polyphenol aggregates consist of polyphenols and proline-rich, so-called haze-active, proteins from the malt. Aggregation and subsequent precipitation of these aggregates is facilitated during the stabilisation phase by cooling the beer to 0 or even -2°C for about 7 to 10 days. According to Narziss (Narziss, L. 1990 Ferment. 3, 54-62), the latter cold stabilisation period is indispensible. After removal of the precipitated matter, non-precipitated polyphenols and/or haze-active proteins are usually removed to prevent the formation of a "chill haze" in the packaged product. To that end residual, non-precipitated polyphenols can be removed by absorption to polyvinylpolypyrrolidone (PVPP) and/or remaining haze-active proteins can be removed by absorption to silica gel. Hereafter the PVPP and/or silica gel, respectively comprising the absored polyphenols and/or haze-active proteins, can be removed together with the removal of any residual yeast, by kieselguhr filtration.

Overall, the above described conventional beer production process takes approximately 20 days. The resulting product is completely clear and stable so that visible changes do not occur during its commercial shelf life.

To increase the flexibility of the brewery and to save capital costs, over the years a lot of work has been done to minimise the fermentation, maturation and stabilisation phases in the brewing process (Narziss, L. 1990 Ferment, 3, 54-62). For example, in the "Pressure Fermentation" process, fermentation and maturation are speeded up by increasing the fermentation and maturation temperatures: usually to temperatures of about 14-18°C. In this approach, fermentation plus maturation are completed within a 7 day period. The whole production process, i.e. including stabilisation, is considerably shortened to slightly over 2 weeks. However, the resulting beers can develop a slightly yeasty taste and overall a less favorable flavor rating.

In the popular alternative, so-called "Cold Fermentation-Warm Maturation" process, beers with a superior taste are obtained by adhering to the conventional, low temperature fermentation process. The desired shorter processing time is achieved by lifting the temperature of the maturation phase to 18 to 22 °C. As a result, the green taste of the fresh beer is overcome within a 2 to 3 day period. Beer stabilisation is achieved by conventional methods. Although the beers obtained have an excellent quality, the energy costs of this process are relatively high.

In yet another approach to speed up beer maturation, green beer is thoroughly centrifuged to remove yeasts and is then passed though bioreactors containing high densities of immobilized yeasts. In combination with an anaerobic heating step to 90 degrees C, all diacetyl is reduced to acetoin within a 1-2 hour period. Also this rapidly matured beer is stabilised with the usual cold storage period and PVPP or silicagel treatment. This bioreactor approach is, however, prone to generate yeasty off-flavors and capital expenditures are likely to be high because advanced technological equipment is required.

Furthermore, the introduction of newly developed enzymes which are added during the beer fermentation phase are beginning to make an impact on beer manufacturing. In a very recent approach, a proline-specific endoprotease is used as an alternative to PVPP or silicagel treatment to prevent chill haze formation (EP 1 326 957). During the beer fermentation, the enzyme selectively hydrolyses the haze-active, proline-rich proteins hereby preventing the precipitation of protein-polyphenol complexes. Reported advantages of this method are the fact that PVPP or silica gel are made redundant because chill haze is effectively prevented by the enzyme. Furthermore the method yields a considerably simplified processing step during this final and most vulnerable stage in beer processing, but has no significant effect on the length of the beer brewing process. In practice, the reactions between PVPP and the polyphenols and/or between silica gel and the haze-active proteins are almost instantaneous. Removal of the PVPP or silica gel treatment, therefore does not yield a shorter beer brewing process. In another enzymatic approach, the enzyme alpha-acetolactate decarboxylase is added to the pitched wort. This addition prevents diacetyl formation during the beer fermentation so that the maturation period, which is primarily aimed at lowering diacetyl levels, can be reduced by 2 to 3 days (cf. US 5,108,925 and US 4,708,875).

Whereas many reports exist on shortening the maturation phase of beer production, so far no reports are available on viable methods to speed up the beer stabilisation phase. However, this stabilisation period typically takes about a week at temperatures around 0°C tieing up considerable capacity and requiring considerable energy input. It would obviously be of great economic importance to further shorten and simplify the process of beer production. Such a shortened and simplified production process would add to the flexibility of brewing plants thereby reducing fixed as well as labor costs.

### Summary of the invention

According to the invention, there is provided use of a proline-specific protease for acceleration of a beer brewing process, preferably by shortening the lagering period.

The invention also provides:
- a method for accelerating a beer brewing process comprising preparing a beer in the presence of a proline-specific protease, wherein the lagering period is shortened; and
- a method for the production of beer comprising fermenting a wort in the presence of a proline-specific protease followed by a maturation phase and stabilisation phase, wherein the stabilisation phase has a duration of less than 7 days.

In addition, the invention provides a beer obtainable by a method of the invention and a bottle, keg or can comprising such a beer

### Brief description of the drawings

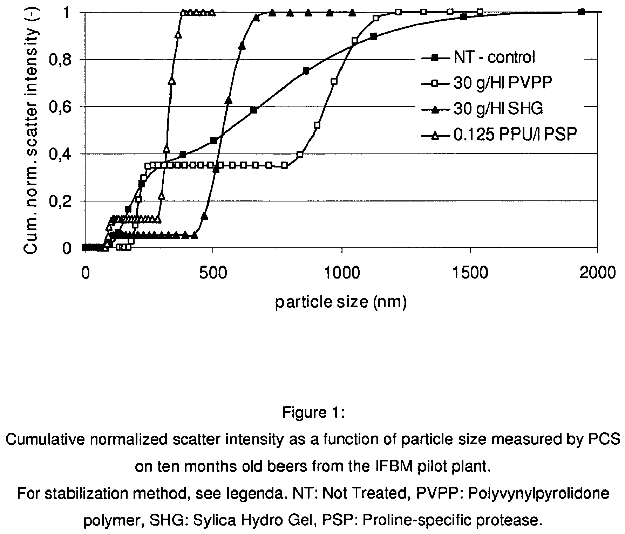

Fig. 1 shows the cumulative normalized scatter intensity as a function of particle size measured by PCS on ten month old beers from the IFBM pilot plant.

### Detailed description of the invention

The present invention relates to the use of a proline-specific protease for accelerating the beer brewing process. In particular, it relates to the use of a proline-specific protease for accelerating the beer brewing process by shortening and simplifying the stabilisation phase of beer production.

The stabilisation phase may be also be carried out at a temperature higher than in conventional brewing processes. In effect, therefore, the invention relates to a process in which the post-fermentation phase of the brewing process is shortened as compared with prior art processes and/or at a higher temperature than prior art processes. Accordingly, the invention also relates to a method for accelerating the beer brewing process comprising carrying out the process in the presence of a proline-specific protease. In such a process, the stabilisation phase may be shorter and/or carried out at a higher temperature as compared with conventional brewing processes.

A large variety of beer brewing processes in used in the world. Typically, however, all these processes basically comprise at least the following steps, or stages corresponding thereto:
- Fermentation
- Maturation
- Stabilisation
- Filtration (some processes do not, however, use filtration)

The process optionally comprises an additional step or steps to further increase stability, for example shelf-life. Any known technique may be used in this regard, for example, the use of a polyvinylpolypyrrolidone (PVPP) and/or silica gel treatment. Typically, such an additional step may be carried out between the stabilisation and the final filtration phase, although such a step might be carried out at some other part of the process.

The specifics of each process step might differ considerably in the different processes, and upon carrying out a beer brewing process, the person skilled in the art will have his own definitions for each phase. To avoid any unclarities, in the context of the present invention, the terms fermentation, maturation, stabilisation, PVPP and silica gel treatment and filtration are intended to be the same as disclosed in the description. Note that in some processes fermentation is also known as primary fermentation. The fermentation phase is the phase in beer brewing intended to ferment available sugars into alcohol by the added yeasts. The maturation phase is also known as secondary fermentation and is intended to convert the undesirable flavour components such as diketones into better tasting components. The stabilisation phase is intended to promote formation of polyphenol-protein aggregates and enable their precipitation. The PVPP and/or silica gel treatment, if used, is intended to respectively remove non-precipitated polyphenols and haze-active proteins to render the packaged product more shelf stable. Finally, the filtration step, if used, is intended to remove precipitated polyphenols and haze active proteins, any residual yeast, the PVPP and/or silica gel prior to packaging.

Generally, during beer brewing a number of parameters may be checked. The end of the fermentation phase may, for example, be determined by measuring the density, of the beer being prepared, to determine the reduction in fermentable extract, such as glucose amount. The end of the fermentation phase is generally considered as the start of the maturation phase. The end of the maturation phase, and thereby the start of the stabilisation phase, may typically be determined by measuring the amount of diacetyl present in the beer. However, in practice this may vary depending on the type of beer desired. For example, in some breweries the maturation phase is considered finished once the vicinal diketone level is below about 0.10 mg/l and in others once the vicinal diketone level is below about 0.05 mg/l. Accordingly, a skilled person will be able to define the end of the maturation phase and beginning of the stabilisation phase according to a desired vicinal diketone level.

In the context of the present invention the start of the stabilisation phase may be defined as the moment where diacetyl levels have been reduced to less than about 0.10 mg/ liter if measured according to EBC method 9.24.1 Vicinal Diketones in Beer: Spectrophotometric Method. In the context of the present invention the end of the stabilisation period is defined as the moment where the beer is subjected to its final filtration step, or in the event that a PVPP and/or silica gel treatment is part of the brewing process, once the beer is contacted with PVPP and/or silica gel. If the process is one in which no filtration is carried out, the end of the stabilisation period is defined as the point at which the beer is packaged.

According to one embodiment of the present invention, the time needed for stabilisation can be shortened to less than about 7 days. Preferably it is less than 6, 5, 4 or 3 days. More preferably, it is shortened to less than 2 or 1 days. Most preferably, it is shortened to such an extent that on production scale, the duration of the stabilisation is reduced to the equivalent of the period required to cool the beer from the maturation phase to the desired temperature, for example the temperature desired for filtration and/or packaging of the beer. This period is conventionally called the cooling period. To shorten the stabilisation period to the time needed to cool the beer down to the desired temperature, is highly advantageous since then the duration of the stabilisation phase only depends on the cooling capacity available.

The beer from the maturation phase is traditionally cooled to a temperature from about -2°C up to and including about 0°C. Surprisingly, it has been found that, upon use of a proline-specific protease according to the invention, the stabilisation phase may not only be shortened, but may also performed at a higher temperature than conventionally used. Traditionally, there are stabilisation processes that are carried out at higher temperatures, but these processes take much more time, for example 6 weeks and more. Therefore, in another embodiment of the invention, the stabilisation period, which may typically be a shortened stabilisation period as defined above, is performed on a beer prefererably cooled to at most about 2°C, more preferably to at most about 3°C, 4°C, 5°C or 6°C or even more preferably to at most about 7 °C or about 8°C and most preferably to the desired temperature used for packaging, ie. for bottle or keg filling.

The desired temperature for packaging can differ from process to process, but is preferably carried out at a temperature up to and including 8°C, preferably around 7°C, in order to obtain the desired level of dissolved carbon dioxide in the beer. In a process where the beer is only cooled down to the temperature required for packaging, considerably less energy is required than in the processes known in the art.

In a preferred embodiment according to the invention, the stabilisation phase is reduced to the period required to cool the beer from the temperature at the end of the maturation phase to the temperature desired for packaging, thereby omitting the conventional cold stabilisation period.

According to the invention, therefore, beer may be cooled to at most about 2°C, more preferably to at most about 3°C, 4°C, 5°C or 6°C, even more preferably to at most about 7°C or 8°C and most preferably to the desired temperature used for packaging. The beer may then be packaged directly, i.e. in this embodiment, the beer is not held for any period of time at the temperature to which it has been cooled.

In the invention, further processing steps may be carried out to achieve additional clarification and/or stability if required. Indeed, if a shortened stabilisation phase is used which corresponds to the period required to cool the beer to the temperature desired for packaging, it may be desirable to treat the beer with PVPP and/or silica gel for example. This may help to ensure extended shelf-life stability.

The industrial implication of this embodiment is that the stabilisation period as such can be omitted from the beer making process thereby saving significant costs and adding to the flexibility of beer producing plants.

The beer stabilised according to the present invention can be further processed to achieve a clarification and stability level that makes the product acceptable for sale. This is generally achieved using an additional treatment step. A filtration step may also be used. This is typically the final step in the brewing process prior to packaging.

Any known technique may be used in this regard. For example, such additional clarification and/or stability treatment may be achieved by using a suitable adsorbant treatment, such as treatment with PVPP, silica gel, gallotannins or immobilized cross-linked insoluble agarose. The use of these treatments and their application to brewing is well know to those skilled in the art. PVPP is typically added at an amount of from about 10 to about 70g/hl. Silica gel is typically added at an amount of from about 10 to about 70g/hl.

Examples of immobilized cross-linked insoluble agaroses include the Combined Stabilisation Systems described in WO97/43401 and US 6,001,406 and the Combined Stabilisation System resin manufactured by GE Health Care Bioscience AB (see Taylor et al. 2006, Use of the Combined Stabilisation System and its impact on beer composition, Proceedings of the Institute of Brewing and Distilling, Asia Pacific Section, Hobart, Australia). Alternatively, or in addition, an enzymatic treatment, such as treatment with papain, could be used to achieve further stability and/or clarity.

The treatments described above may be used in an immobilized form, for example immobilized PVPP particles.

All of the additional treatments set out above may be used in conjunction with the stabilisation procedure of the invention. A combination of the additional treatments, for example two, three, four or all of such additional treatments, may be used in conjunction with the stabilisation procedure of the invention.

Conveniently, such additional clarification and/or stability treatment may be carried out once a beer has been stabilized according to the invention. The invention does, however, also relate to processes in which an additional clarification and/or stability treatment is carried out at some other point in the process or simultaneously with stabilisation according to the invention.

In a preferred embodiment of the invention, the proline-specific endoprotease as disclosed in EP-A-1326957 is used, thereby optionally making the PVPP and silica treatment superfluous and making the inherent costs for equipment for PVPP regeneration and the labor costs connected with the operation of this equipment redundant. Additionally the natural antioxidant potential of beer is increased and the marginal colloidal stability of many PVPP or silica gel treated beers is improved. Furthermore, the risk of oxygen exposure of the beverage is drastically reduced and waste streams are minimized.

In a preferred embodiment of the present invention, this shortening or even omission of the stabilisation phase is combined with a shortening of the maturation phase (the phase primarily aimed at converting diacetyl to acetoin). In the context of the present invention the start of the maturation phase is hereinafter defined as the moment where the apparent degree of attenuation reaches from about 70% to about 90%, more preferably from about 77 to about 84% as estimated using a density measurement of the beer. The density of the beer can be measured by a pycnometer or a density meter (EBC Methods 8.2.1 and 8.2.2 respectively) and the attenuation limit is determined according to EBC method 8.6.1 or 8.6.2 (Fermentability, Attenuation Limit of wort). In the context of the present invention, the end of the maturation phase may be defined as the as the moment where the diacetyl levels have been reduced to less than about 0.10 mg/ liter if measured according to EBC method 9.24.1 Vicinal Diketones in Beer: Spectrophotometric Method. The maturation phase may be shortened by methods known in the art. For example, maturation may be accelerated by using high temperatures as described for the "Pressure Fermentation" and the "Cold Fermentation-Warm Maturation" processes (Narziss, L. 1990 Ferment, 3, 54-62). Maturation may also be shortened by the use of immobilised yeasts in a bioreactor approach or by employing acetolactate decarboxylase activity (ALDC: EC 4.1.1.5.; see for instance US 5,108,925 or US 4,708,875). Using one or a combination of these methods, maturation may be limited to two days, one day or even less than one day as feasible using a bioreactor operated at a high temperature.

In the context of the present invention, the phase which incorporates both the maturation and stabilisation phases of beer production is called the lagering period.

Accordingly, the invention provides use of a proline-specific protease for accelerating a beer brewing process. Typically, the acceleration occurs by way of a shortening of the post-fermentation period, for example by shortening the duration of the lagering period, preferably by reducing the stabilisation period.

The terms "accelerating" and "shortening" in this context are intended to indicate a beer brewing process which takes less time to carry out as compared with an equivalent process where a proline-specific protease is not used. The acceleration generally occurs as a consequence of a shortened post-fermentation period, i.e. that period may be carried out for less time when using a proline-specific protease as compared with an equivalent process which does not use such an enzyme.

If the use of the invention is combined with measures to shorten the maturation phase, the whole lagering period may be restricted to less than 8, 7 or 6 days. Preferably, it is restricted to less than 5, 4, or 3 days. More preferably, it is restricted to less than 2 days. Even more preferably, the whole lagering period is restricted to less than 24 hours.

In a preferred embodiment of the present invention, a lagering period which is restricted to 4 days or less may be achieved by combining a proline-specific protease with an acetolactate decarboxylase, for example an alpha acetolactate decarboxylase.

In a preferred embodiment, the proline-specific protease and, optionally, the acetolactate decarboxylase are added to the primary fermentation. After this primary fermentation, the green beer may be subjected to a rapid maturation procedure and then, after the diacetyl levels have been reduced to less than about 0.10 mg/ liter, the beer may be immediately cooled down to about 2°C, preferably to about 5°C, more preferably to about 7°C and most preferably to a temperature suitable for packaging and then, optionally, filtered to obtain a clarified beer, ready for bottling. In this way, an acceleration of the post-fermentation phase, and also of the overall brewing process may be achieved.

The advantages of the process according to the invention apply to top- as well as to the bottom fermented lager beers. Top fermented beers ferment and mature very rapidly but, like bottom fermented beers, they require lengthy cold storage periods to remove polyphenol-protein complexes. The advantages of the process according to the invention particularly apply to bottom fermented lager beers. The use and method of the invention may also be applied to beers in which spontaneous fermentation has taken place.

In another aspect, the present invention relates to a method wherein the proline-specific protease is used according to the invention. That is to say, the invention provides a method for accelerating a beer brewing process, preferably by reducing the duration of the lagering period (such as by reducing the duration of stablisation period), which method comprises carrying out the said beer brewing process in the presence of a proline-specific protease. The lagering phase, for example the stabilisation phase may be carried out at temperature higher than is typically used in conventional beer brewing processes. Typically, the duration of the stabilisation phase will be the time required to cool the beer from the maturation phase to the packaging temperature.

In the context of the present invention, the words peptide and protein are used interchangeably. In the context of the present invention, the words "haze", "cloudiness" and "turbidity" are also used interchangeably.

To determine the moment where the apparent degree of attenuation reaches from about 77 to about 84%, density measurement of the beer is used. EBC method 9.24.1 Vicinal Diketones in Beer: Spectrophotometric Method is used to quantify diacetyl levels. Note that EBC method 9.24.1 is designed to measure diketones in general, however since diacetyl is by far the main component of the diketones, therefore in the context of the present invention the outcome of this measurement method is regarded as indicative for the diacetyl level. The diacetyl level can alternatively be measured with a gas chromatograph using EBC method 9.24.2 Vicinal Diketones in beer. To quantify the amount of haze in a beverage, a turbidimeter may be used. In a turbidimeter the amount of light is measured that is scattered at a predescribed angle relative to the direction of the incident light beam. Turbidity measurements are known to be very suitable for the measurement of haze formed as the result of protein-polyphenol interactions.

A polyphenol is defined as a compound having a chemical structure which structure contains at least two aromatic rings substituted with at least one hydroxyl group or having a chemical structure which contains at least one aromatic ring substituted with at least two hydroxyl groups. Examples of polyphenols are tannins and flavonoids, which include for example catechins, flavonols and anthocyanins.

The term "beer" as used herein is intended to cover at least beer prepared from mashes prepared from unmalted cereals as well as all mashes prepared from malted cereals, and all mashes prepared from a mixture of malted and unmalted cereals. The term "beer" covers bottom fermented but also top fermented beers as well as beers prepared with adjuncts, and beers with all possible alcohol contents.

The amount of proline-specific protease that is added during fermentation may vary depending on the levels of malt used and the type of fermentation carried out. In a preferred embodiment of the use and/or method according to the invention, from about 7.5 to about 15 units (PPU), for example from about 10 to about 12.5 units (PPU) of proline-specific endoprotease activity is added per hectoliter of a 100% malt beer at for example about 12 degrees Plato. A maximum amount of proline-specific protease activity to be added cannot be specified. The maximum amount is for example dependent on the desired amount of haze reduction or prevention, the composition of the beer on the pH at which the protease has its maximum activity. The unit definition of the enzyme is provided in the Materials & Methods section of this application.

The proline-specific protease may be added at different stages during the preparation of a beer. Addition of the enzyme at the beginning of the fermentation yields the best possible results. However, the enzyme may be added to a mash or to a fermented beer before haze has been formed.

In this text, the terms prolyl-specific protease, proline-specific protease, proline-specific endoprotease, proline-specific endopeptidase and protease having a prolyl-specific activity or similar expressions are used interchangeably.

The proline-specific protease may be used in the invention in an isolated or purified form. By "isolated" or "purified" is intended a proline-specific protease removed from its native environment. For example, recombinantly produced proline-specific protease expressed in host cells are considered isolated for the purpose of the invention as are native or recombinant polypeptides which have been substantially purifiried by any suitable technique such as, for example, the single-step purification method disclosed in Smith and Johnson, Gene 67:31-40 (1988).

A proline-specific protease suitable for use in the invention may be recovered from recombinant cell cultures by methods well-known to those skilled in the art, including for example ammonium sulfate or ethanol preciptation, acid extraction and chromatographic methods such as high performance liquid chromatography (HPLC).

A proline-specific protease suitable for use in the invention may be a naturally-purified product, a product or a chemical synthesis, a product produced by a recombinant technique from a prokaryotic or eukaryotic host, including, for example. Bacterial, yeast, fungal, higher plant, insect and mammalian cells.

It will be understood though that the protease may be be mixed with carriers or diluents which will not interfere with the intended purpose of the enzyme and still be regarded as isolated. A proline-specific protease suitable for use in the invention may also be in a more substantially purified form. Accordingly, the proline-specific protease may be comprised in a preparation in which more than 70%, for example more than 80%, 90%, 95%, 98% or 99% of the proteins in the preparation is a proline-specific protease.

Typically, the proline-specific protease may be in a form free from or substantially free from any other protease.

A proline-specific protease suitable for use in the invention may be used in an immobilized form so that large quantities of protein containing liquids can be treated. Ways to select appropriate support materials and suitable immobilization methods have been extenvsivelty described in the literature, for example in "Immobilization of Enzymes and Cells" (ed. Gordon F. Bickerstaff; ISBN 0-89603-386-4).

In the context of the present invention, a proline-specific protease is defined as an protease that cuts proteins or peptides at places where the protein or peptide contains a proline residue in its chain. Preferably, a proline-specific protease is an endoprotease that "cuts" (hydrolyses) proteins or peptides at places where the protein or peptide contains a proline residue. In the method according to the invention, a proline-specific endoprotease is preferably used that hydrolyses the peptide bond at the carboxyterminal end of proline residues. Examples of such enzymes are prolyl oligopeptidases (EC 3.4.21.26) as well as the Aspergillus niger derived prolyl endoprotease reported in J. Agic Food Chem, Vol 53 (20), 7950-7957, 2005) and proline-specific dipeptidyl peptidases such as DPP IV (EC 3.4.14.5). A proline-specific endoprotease that cuts proline-residues at their NH₂-terminus is for example described in a publication in Nature of 15 January 1998, Vol.391, p.301-304.

As is typical for enzyme activities, the activity of proline-specific endoproteases is dependent on the pH. Typically, a proline-specific endoprotease is used in the invention which has a maximum prolyl-specific activity at a pH which corresponds to the pH of the beer (or mash or wort etc.) to which it is added. In a preferred embodiment of the method according to the invention, a protease with an acidic pH optimum, i.e. with a pH optimum of 6.0 or lower - for example pH 5, 4, or 3 - is added to the primary beer fermentation. In a more preferred embodiment of the use and/or method according to the invention, a protease with an acidic pH optimum and which is actively secreted by a food grade microorganism into the fermentation broth is added to the beer fermentation.

Proline-specific proteases are widely found in animals and plants, but their presence in microorganisms appears to be limited. To date, proline-specific proteases have been identified in species of *Aspergillus* (EP 0 522 428), *Flavobacterium* (EP 0 967 285) and *Aeromonas* (J.Biochem.113, 790-796), *Xanthomonas* and *Bacteroides.* Though the proline-specific enzymes from most of these organisms are active around pH 8, the *Aspergillus* enzyme is optimally active around pH 5. The proline-specific protease of the invention may be isolated from one of the above-mentioned microbial species, particularly from a species of *Aspergillus.* Preferably, the proline-specific endoprotease is isolated from a strain of *Aspergillus niger.* More preferably, the proline-specific endoprotease is isolated from an *Aspergillus niger* host engineered to overexpress a gene encoding a proline-specific endoprotease, although other hosts, such as E. *coli* are suitable expression vectors. For example, the cloning and overproduction of the *Flavobacterium* derived proline-specific endoprotease in, amongst others, *E.coli* has made certain proline-specific endoproteases available in a pure form. An example of such an overproducing construct is provided in the World Journal of Microbiology & Biotechnology, Vol 11, pp 209-212. An *Aspergillus niger* host is preferably used to produce a non-recombinant self-construct utilizing *A. niger* promoters to drive the expression of a gene encoding an *A. niger* proline-specific endoprotease.

Most preferably the proline-specific endoprotease is the endoprotease as disclosed in EP-A-1326957, which protease is herein incorporated by reference. In EP-A-1326957, also the use of the proline specific endoprotease in beer brewing is disclosed. In that document, however, only the use to reduce haze in beverages is disclosed. It has not been disclosed that the proline-specific endoprotease could be used to accelerate the beer brewing process as such. Furthermore, to the person skilled in the art the PVPP and/or silica gel treatment are known to be instantaneous. No methods have been disclosed wherein the lagering period or the stabilisation period was shortened. In case one would use a conventional process without a proper stabilisation phase, it would be apparent to the person skilled in the art that this would result in beers of bad quality, for example with respect to colloidal stability and clarity. It was therefore surprising that, upon the use of a proline-specific endoprotease in beer brewing, the process could be shortened without detrimental effects on the beer quality.

The acetolactate decarboxylase activity added during fermentation may vary between limits known to the person skilled in the art. An indication of the activities required is provided in a paper by Hannemann (MBAA TQ, Vol 39, no 3, 2002, 149-155).

Modern breweries strive to remove all bulk powders like PVPP, silicagel or kieselguhr from the beer brewing process. In this approach, the final kieselguhr filtration is replaced by a membrane filtration or crossflow filtration. According to another embodiment of the invention, the beer production process according to the invention may be carried out without the use of any bulk powders like PVPP or silica hydrogel or kieselguhr for filtration. Accordingly, beer prepared according to the use and/or method of the invention may be free from, or substantially free from, PVPP and/or silica hydrogel and/or kieselguhr.

Using the method according to the invention, the beer may be clarified using filter techniques such as crossflow filtration. Accordingly, the invention covers a process in which a beer is prepared using a proline-specific protease in combination with a crossflow membrane filter.

The present invention relates to a beer brewing method, whereby a proline-specific protease is used, whereby the stabilisation period can be shortened with respect to a brewing process wherein no proline-specific protease is used, whilst at least maintaining the same properties with respect to the visual clarity of the beer according to EBC 9.29 terminology for visual determination.

The invention also relates to beer obtained by the method according to the invention. Usually clarity of beers can be judged in various ways. EBC method 9.29 describes how haze in beers is measured. EBC units are measured in turbidimeters under a 90 degree angle. Alternatively, visual assessment can be done. According to the EBC method 9.29, ie. when measuring the EBC units under a 90 degree scatter angle, beer having a turbidity below 0.5 EBC units, will be visually assessed as "brilliant". In case the turbidity of the beer is between about 0.5 and 1.0 EBC units, the beer will be visually assessed as "very clear", between 2 and 1 EBC units "very slightly hazy", between 2 and 4 EBC units "hazy" and above 8 EBC units "very hazy". Of course no exact equivalence must be expected between different haze scales or between visual observations made in different laboratories, but a large discrepancy cannot be expected. Moreover, in the end the visual inspection as done by the consumer, will be important from a commercial point of view. Therefore, it is generally accepted that a beer should be (almost) brilliant to be commercially acceptable, ie have a EBC unit value measured under 90 degree scatter of at most 1.

Surprisingly, it has been found that for the beer prepared with the method according to the invention, the EBC unit value did not correspond to the visual assessment by a trained panel. It is presumed that the higher amounts of protein and polyphenols still present in the beer result in higher 90 degree scatter values, but cannot be detected with the eye. As shown in the Examples, it has been found possible to have a visual assessment of "brilliant" for a beer having an EBC unit value higher than 1 and also a visual assessment of "almost brilliant" for a beer having an EBC unit value higher than 2, ranging up to even 8. That is to say, beer obtained according to the method of the invention has a different (physo)chemical composition to conventionally brewed beers.

This has been confirmed using particle analysis of beers stabilized by different methods. This showed that beers stabilized according to the invention comprises particles which are smaller on average than those identified in beers stabilized using PVPP or silica hydrogel.

Therefore, another aspect of the invention is a beer having a turbidity higher than 1 EBC unit, preferably equal to or higher than 1.25 and most preferably equal to or higher than 1.5, whilst the visual assesment by a trained panel using EBC terminology will scale the beer as "brilliant" immediately after brewing and measured at 1°C. In another embodiment the invention relates to a beer having a turbidity higher than 2 EBC units, preferably higher than 3, more preferably higher than 4, even more preferably higher than 6 and most preferably higher than 8 EBC units, whilst the visual assessment by a trained panel using EBC terminology will scale the beer as "almost brilliant" measured at 1°C after at least 3 months of storage of the beer at ambient temperature, preferably at least 6 months storage of the beer or most preferably 9 months storage of the beer at ambient temperature.

The beer obtained according to the use and/or method of the invention will typically be packaged. Any suitable packaging may be used, for example a bottle, a keg or a can. Beer prepared according to the use and/or method of the invention may also be packaged in a bulk-tank. Accordingly, the invention provides a packaging comprising the beer obtained according to the use and/or method of the invention, for example a bottle, a keg or a can comprising such a beer

Hereafter, the invention is illustrated by the following non-limiting Examples.

### MATERIALS & METHODS

### Activity measurements of proline- specific enzymes

The activity of proline-specific endoproteases having pH optima below pH 6.0 are tested on the synthetic peptide Z-Gly-Pro-pNA at 37°C in a citrate/disodium phosphate buffer pH 4.6. The activity of proline-specific endoproteases having neutral pH optima are tested on the synthetic peptide Z-Gly-Pro-pNA at 37°C in a phosphate buffer pH 7.0.

The activity of dipeptidyl dipeptidases having a pH optimum below pH 6.0 are tested on the synthetic peptide Gly-Pro-pNA at 37°C in a citrate/disodium phosphate buffer pH 4.6.

The activity of dipeptidyl dipeptidases having pH optimum below pH 6.0 are tested on the synthetic peptide Gly-Pro-pNA at 37°C in a phosphate buffer pH 7.0.

The reaction products of all proline-specific proteases are monitored spectrophotometrically at 405 nM. One unit (1PPU) is defined as the quantity of enzyme that liberates 1 µmol of p-nitroanilide per minute under these test conditions.

### Analyses during the brewing process

The various analyses were carried out in accordance with the 2004 edition of "Analytica - EBC" (Fachverlag Hans Carl, Nurnberg, Germany), unless indicated otherwise. Malt analyses were carried out according to the following EBC methods 4-2, 4-5-1, 4-9-1, 4-3-1, 4-18, 8-13-2, 4-8, 4-15 and 4-14. Mashing was monitored by measuring the saccharification rate and the mash filtration. The worts were analyzed according to EBC 4-5-1, 4-7-2, 4-11 as well as free amino nitrogen (EBC method 8.10), total nitrogen (either by EBC method 8.9.1 - Kjeldahl - or 8.9.2 - Dumas Combustion method), pH and total wort polyphenol (EBC method 8.12). The various fermentation experiments were followed by temperature, extract decrease and pressure. Yeast population was determined at pitching, first day of fermentation and before beer filtration. Diacetyl was measured by gas chromatography during fermentation, maturation and just before filtration. The beer filtration was monitored by yeast counts before and after filtration, by the relevant pressure drops and flow rates. Dissolved oxygen was measured in the carbonated water, in beer the filtration inlet and outlet and in the bottled beer. The various beer analyses included EBC 9.4, 9.6, 9.7, 9.8, 9.11,, 9.24.1, 9.24.2, 9.29, 9.30, 9.35,9.37, Ross & Clark and NIBEM (9.42) methods for head retention values, trans-2-nonenal, and reducing power. Haze development was followed using various haze measurements at various temperatures (see Example 2) at 25 and 90 degrees scatter angles using the VOS ROTA 90/25 Hazemeter (Haffmans, Venlo, The Netherlands). Forcing tests were carried out according to the EBC 9.30 predictive shelf-life test.

### The brewing process

All beer brewing experiments were carried out on a 20 hl scale with 300 kg of pilsen malt (Heineken A type) and 950 I of water for the mashing. The brewing incorporated a mashing process with the following temerature regime: 45°C for 20 minutes, 45 to 64 °C in 20 minutes, at 64 °C for 15 minutes, from 64 to 76 °C in 12 minutes, at 76°C for 25 minutes and finally from 76 to 78°C in 5 minutes followed by mash filtration. For sparging hot water was used. Boiling was for 90 minutes and hop was added in the form of hop pellets. Cold stabilisation was carried out under conditions as specified in the individual Examples. The beer was filtered using a plate filter coated with a coarse- and a fine-grade pre-coat. The fine-grade pre-coat was also used as body feed. For bottling a Carbofill filler was used. The bottles were pasteurized during 15 minutes at 60°C.

### EXAMPLES

### Example 1

### Pilot scale beer production

At the 20 hl semi-industrial pilot Brewery at IFBM (Institute Français de la Brasserie et de la Malterie, Vandoeuvre-les-Nancy, France) two pure malt worts were brewed under identical conditions. During fermentation, to one of these worts a proline-specific protease (Brewers Clarex from DSM Food Specialities, Delft, The Netherlands, containing 5PPU/gram product) was added in a concentration of 10 PPU/hectoliter wort, as well as acetolactate decarboxylase (Maturex L from NOVO, Bagsvaerd, Denmark, containing 1,500 ADU/gram product) in a concentration of 4500 ADU/ hectoliter of wort at the beginning of fermentation. This fermentation was designated "trial fermentation". The other wort was fermented as such, and was designated "control fermentation".

### Brewing

The brews were produced from 300 kg barley malt and hop pellets. Mashing conditions were a water:grist ratio of 4:1 (vol/wt), pH 5.6. The mashing diagram included a first step at 45°C for 20 minutes, a second step at 64 °C for 15 minutes, a third step at 74°C for 30 minutes, and finally a mash-off at 78°C for 5 minutes. The heating rates were 1 °C/minute each time. After mashing-off, the mash was filtered in a Lauter tun; first wort recycling and hot (78°C) spargings at pH 5.6 were applied. The resulting worts were boiled for 90 minutes, after which good trub separations were performed with a whirlpool.

### Fermentation

The fermentations were carried out with the bottom yeast strain Rh as purchased from VLB (Berlin, Germany) pitching with 17.10⁶ viable cells/ml of the 12°C Plato wort. The fermentation process was conducted at 12°C until 5°C Plato (3 days) and was then continued at 14°C till the end of fermentation (3 days).

### Post-fermentation

At the end of fermentation, the control fermentation was held at 14°C for three more days to reduce the diacetyl levels. After this maturation period, the beer was cooled to 0°C in one day and the beer was stabilized for 5 days at 0°C prior to single-use PVPP treatment at a dosage 30 g/HI, beer filtration with diatomaceous earth, and bottling.

For the trial fermentation, however, no maturation period was applied. In addition, the stabilisation period was restricted to the period required to cool the beer to 4°C. Directly after the end of fermentation, the beer was cooled down to 4°C during a 4-day period, following a linear temperature decrease. Because Brewers Clarex was used during fermentation, no beer stabilizing agents (such as PVPP or silica gel) were added. Instead, after cooling the beers were directly filtered with diatomaceous earth, and subsequently bottled. Standard beer parameters (e.g. pH, color, bitterness, ethanol, etc.) were assayed in accordance with the methods established by the European Brewery Convention (EBC), which are described in their "Analytica - EBC".

### Results

Generally, standard beer analysis indicated that the beer parameters were similar for both control and the trial fermentation. Dissolved oxygen levels were comparable in both trials and below 0.2 mg/l. Initial haze levels were comparable for both beers, whereas the trial beer has significantly lower values in the alcohol Chill haze test according to L. Chapon (EBC method 9.41). Additionally, during the beer forcing test "Prediction of the Shelf-Life of Beer" (EBC method 9.30), the trial beer performed better than the beer from the control fermentation.

The level of vicinal diketones from the beers from both the trial and control fermentations were below 0.10 mg/liter (measured with EBC method 9.24.1 Vicinal Diketones in Beer: Spectrophotometric Method). Sensory analysis was performed by a trained panel (consisting of eight persons) at IFBM (Vandoeuvre-les-Nancy, France) in a standardized procedure, in which after tasting the sample, assessors awarded an intensity score for taste attributes. Results were collected to form a sensory profile of the sample. The tests were done in accordance with the guidelines for sensory analysis, described in Chapter 13 of the 2004 edition of "Analytica - EBC". No significant taste differences could be observed by the taste panel between the beer from the control and trial fermentation.

### Conclusion

This example demonstrates that by the application of a proline-specific protease and aceto lactate decarboxylase a beer could be produced which has standard beer parameters (measured with methods established by the European Brewery Convention (EBC)) which are similar to the parameters of the reference beer and which shows no significant taste differences in comparison to the reference beer. In additon, the use according to the invention leads to a time-saving process, because a lagering period of 9 days (control) could be reduced to 4 days. Furthermore, significant energy savings could be achieved, by cooling to 4°C during 4 days, instead of cooling to 0°C in one day and maintaining this temperature for another 5 days.

### Example 2

### Shortening the cold stabilization period and its effects on the clarity of enzyme stabilized, 100% malt beers

Brewing trials were carried out to evaluate the effect of a proline-specific endoprotease on 100% malt beers upon using shortened cold stabilization periods. To that end, six different worts were prepared and fermented according to exactly the same protocol (see Materials & Methods). To five fermenters, the proline-specific protease from *Aspergillus niger* was added at a concentration of 0.125 PPU/liter (see Materials & Methods for activity definition) and in all five fermentations, the enzyme was added to the cold wort, before pitching. The sixth fermentation served as a reference and no proline-specific enzyme was added. All fermenters were pitched with fresh lager yeast (approx 17 million cells/ ml wort) and fermentation took place at 12°C till 5 plato followed by a temperature increase till 14°C for the removal of vicinal diketones such as diacetyl. At the end of maturation (in this case nine days after the beginning of fermentation) the beer was cooled down to minus 1 °C. The matured beer from all fermenters with the enzyme added, was kept for various periods at minus 1°C and was then kieselguhr filtered. Four batches to which proline-specific enzyme had been added, were filtered as such. The fifth batch with added proline-specific protease, was treated in-line with single use PVPP (40 g/ hi; injected to the beer filter) and then filtered.

The matured beer from the fermenter with no enzyme added, was also cooled to minus 1°C and subjected to a 'classical' stabilization protocol i.e. the beer was kept at minus 1 °C for 9 days followed by the same in-line treatment with 40g/hl single-use PVPP and, subsequently, kieselguhr filtered. After filling, bottles were pasteurised at 15 PU and stored at 20°C.

After a storage period of six weeks, the various beers were subjected to haze measurements at 20°C and at 1 °C. To that end, the beer was preincubated for 24 hours at measurement temperature, i.e. either 1 or 20°C and then beer haze was measured at this temperature. Haze readings were recorded using a turbidimeter measuring at 90 and 25 degree scatter. The 90 and 25 degree scatter data obtained with this turbidimeter (a Haffmans VOS ROTA 90/25) are shown in Table 1 as "H90" and "H25" values, respectively, together with the relevant measurement temperature. Prior to these haze measurements, bottles were shaken to homogenize and left long enough for gas bubbles to disappear. Multiple measurements were carried out to ensure that gas bubbles did not influence the reading. Apart from these haze measurements, visual and taste assessments were carried out using a trained panel (n=5).

**Table 1.**

| | Batch 1 | Batch 2 | Batch 3 | Batch 4 | Batch 5 | Reference |
|---|---|---|---|---|---|---|
| Number of stabilisation days at -1 °C | 0 | 1 | 3 | 5 | 0 | 9 |
| Prol spec Enzyme | yes | yes | yes | yes | yes | no |
| PVPP (40 g/hl) | no | no | no | no | yes | yes |
| Actual haze (H25) at 20 °C | 0.30 | 0.31 | 0.32 | 0.28 | 0.28 | 0.27 |
| Actual haze (H25) at 1 °C | 0.34 | 0.34 | 0.37 | 0.32 | 0.30 | 0.28 |
| Actual haze (H90) at 20 °C | 0.48 | 0.59 | 1.06 | 0.83 | 0.48 | 0.48 |
| Actual Haze (H90) at 1 °C | 0.52 | 0.66 | 1.17 | 0.90 | 0.53 | 0.49 |
| Forcing test according to EBC 9.30 (H25) | 0.70 | 0.35 | 0.33 | 0.27 | 0.22 | 0.22 |
| Forcing test EBC 9.30 (H90) | 1.0 | 0.75 | 1.26 | 0.92 | 0.52 | 0.48 |
| Visual assessment at 8°C at six weeks after bottling (terminology EBC 9.29) | brilliant | brilliant | brilliant | brilliant | brilliant | brilliant |
| Taste (3 weeks after bottling) | good | good | good | good | good | good |

According to a visual inspection, all bottled beers, i.e. including the enzyme-treated beer that was not subjected to any cold stabilization period, were completely clear after cooling for 24 hours to 8°C after a six week storage period at 20°C.

Quite surprisingly, these visual haze assessments were not completely in line with the different haze readings obtained. According to the EBC standard, beers measured according to the EBC 9.29 method (measured at 90 degrees scatter angle) and yielding values between 1.0 and 2.0 EBC are "very slightly hazy". Above 2.0 EBC, beers start to become " slightly hazy". The 90 degree scatter readings yielded values up to 1.26 EBC (see Table 1), and yet all of these beers were rated visually as "brilliant". This discrepancy is adressed in Example 3.

Also noteworthy is that, according to the expert taste panel, none of the beers produced showed an aberrant taste profile.

### Example 3

### Turbidity measurements and haze formation

Three 20 HI fermentations were conducted according to the protocol described in Example 2 for a 12 Plato 100% malt wort. The first fermentation (reference) was carried out without addition of a proline-specific protease during the fermentation. To the two other fermentations, the proline-specific protease from A. niger was added at pitching in concentrations of 0.125 and 0.20 PPU/I respectively. At the end of maturation (in this case nine days after the beginning of fermentation), all three beers were cold stabilized for 5 days at 1 °C, after which yeast and cold precipitates were removed. In neither case was a PVPP or a silicagel treatment applied. All three beers were filtered over a kieselguhr-filter, which had a coarse and a fine grade precoat. Finally, the beers were bottled and pasteurized at 15 PU and then stored at ambient temperature for shelf life studies.

After various storage periods, the beer was preincubated for 24 hours at 1 °C and then beer haze was measured at this temperature at 90 degree scatter angle. Prior to measurement, bottles were shaken to homogenize and left long enough for gas bubbles to disappear. Multiple measurements were carried out to ensure that gas bubbles did not influence the reading. The data obtained are summarized in table 2.

**Table 2.**

| Months of storage at 20 °C | 0 | 3 | 6 | 9 |
|---|---|---|---|---|
| | H90 (EBC) values measured at 1 °C | | | |
| Reference (no enzyme) | 7.2 | 24.8 | 20.7 | 21.8 |
| Fermentation+enzyme (0.125 PPU/I) | 1.5 | 4.6 | 7.5 | 6.9 |
| Fermentation+enzyme (0.20 PPU/I) | 1.3 | 4.0 | 6.9 | 5.2 |

Together with the turbidity measurements, the beers were visually inspected by a trained panel (n=5) and judged using the EBC 9.29 terminology.

As expected, the non-enzyme treated reference beers showed high H90 EBC values. In line with these high H90 values, the visual inspection rated all samples at t= 0, 3, 6 and 9 months as "very hazy" (ratings used the terminology specified in EBC method 9.29).

Of the enzyme-treated beers, only the fresh (t=0) samples showed low H90 values (1. 5 and 1.3 EBC respectively). In contrast with our expectations, the visual panel readings for the fresh, enzyme-treated beers (t=0 months) were "brilliant" whereas, on the basis of the H90 values as obtained, a rating of "very slightly hazy" was expected.

Enzyme-treated samples stored for longer periods, all showed H90 values of 4 EBC or higher. Surprisingly, the visual ratings obtained from the two enzyme-treated beers after 3, 6 and 9 months of storage, were rated "almost brilliant" rather than "hazy" as to be expected from their H90 values.

Therefore, we have to conclude that, as a result of the use of the proline-specific protease in the beer fermentation process, the instrumental H90 scatter values are not indicative for the visual haze perception, nor for the colloidal stability of packaged beer according to the invention. Furthermore, we have to conclude that the beer according to the invention is different from beers known in the art.

### Example 4

### Particle size analysis of beers stabilized by different methods

In the IFBM (Institute Français de la Brasserie et de la Malterie, Vandoeuvre-les-Nancy, France) semi-industrial pilot brewery, four fermentations were conducted according to the protocol described in Example 2 for a 12 Plato 100 malt wort. To one of the fermentations the proline-specific protease from A. niger was added at pitching at a concentration 0.125 PPU/I. To the other three fermentations, no proline-specific protease was added. At the end of maturation (in this case nine days after the beginning of fermentation), all beers were cold stabilized for 5 days at 1 °C, after which yeast and cold precipitates were removed. One of the three beers that were not stabilized with the proline-specific protease was then stabilized with 30 g/HI single-use PVPP (injected inline to the beer filtration). Another beer to which no proline-specific protease was added, was stabilized with 30 g/HI silica hydrogel (injected inline to the beer filtration). The beer treated with the proline-specific protease from A. niger and the remaining "untreated" control beer were filtered as such, i.e. without the addition of PVPP or silica hydrogel. All four beers were filtered over a kieselguhr-filter, which had a coarse and a fine grade precoat, and a kieselguhr body feed was added during filtration in all cases. Finally, the beers were bottled and pasteurized at 15 PU and then stored at ambient temperature. During the process, the oxygen levels were carefully minimized, and were found to be comparable for all four beers.

After approximately 10 months of storage at ambient temperature, the particle size distribution of the four beers was studied at Brewing Research International (BRI, Nutfield, United Kingdom) using Photon Correlation Spectroscopy (PCS) at 6 °C. This technique does not count particles as such, but provides a light scatter reading and is able to attribute proportions of that scatter to specific size classes of particles, hereby allowing comparison of the particle size distributions of the four beer samples.

The cumulative normalized scatter intensity is plotted in Figure 1. Figure 1 indicates that in the "untreatred" control beer, all scattered light results from particles smaller than 2000 nanometer. For the PVPP and silica hydrogel stabilized beers, all particles that scatter light are smaller than approximately 1250 and 750 nm, respectively. However, the particles in the proline-specific protease treated beer are much smaller on average: no particles were found larger than 385 nm. For comparison the graphs in Figure 1 were quantified by two variables:
- d50, the particle diameter at which 50% of the light scatter is from particles smaller than that diameter, and:
- d90, the particle diameter at which 90% of the light scatter is from particles smaller than that diameter.

The latter data are provided in Table 3.

**Table 3: d50 And d90 values (see text for explanation) for iFBM beer samples with different stabilization. NT: Not Treated, PVPP: Polyvynylpyrolidone polymer, SHG: Sylica Hydro Gel, PSP: Proline-specific protease.**

| *Treatment* | ***Temperature** (°C)* | ***d₅₀** (nm)* | ***d₉₀ (nm)*** |
|---|---|---|---|
| *NT-control* | 6 | *895* | *1073* |
| *30g*/*hl PVPP* | 6 | *556* | *1141* |
| *30g*/*hl SHG* | 6 | *537* | *629* |
| *0.125 PPU*/*I PSP* | 6 | *325* | *361* |

Both the d50 and d90 values in Table 3 indicate that the particles generating scatter in the beer with the proline-specific protease are substantially smaller than in the PVPP or silica hydrogel stabilized beers. This is in line with our hypothesis, that the hydrolysis of the haze-active proteins by the proline-specific protease, prevents or reduces protein-polyphenol complex formation during aging. We conclude that, as a result of the action of the proline specific protease, the haze-active proteins are hydrolyzed so that the complexes between proteins and polyphenols remain smaller during storage, thus leading to the observed discrepancy between the visual haze assessment by expert panels and the relatively high instrumental 90 degree scatter readings. Again, we conclude that the beer obtainable using the method of the invention is different from beers known in the art.

### Example 5

### Shortening the cold stabilization period and its effects on haze development in 100% malt beers after storage of 4 and 6 months at ambient temperature

In this Example data on the long term stability of the beers described in Example 2 is provided. The bottled and pasteurized beers were stored at ambient temperature (about 20 degrees C) for a period up to 6 months. After 4 and 6 months of storage, the beers (Batches 1, 2, 3, 4 and 5 plus the Reference as listed in Table 1) were analyzed using turbidity measurements and visual evaluation also described in Example 2. The data obtained are presented in Table 4.

**Table 4**

| | Batch 1 | Batch 2 | Batch 3 | Batch 4 | Batch 5 | Reference |
|---|---|---|---|---|---|---|
| Number of stabilisation days at -1°C | 0 | 1 | 3 | 5 | 0 | 9 |
| Prol spec enzyme | yes | yes | yes | yes | Yes | no |
| PVPP (40 g/hl) | no | no | no | no | Yes | yes |

| Data obtained after 4 months storage at ambient temperature | | | | | | |
|---|---|---|---|---|---|---|
| Actual haze (H25) At 20°C | 0.60 | 0.58 | 0.51 | 0.38 | 0.36 | 0.36 |
| Actual haze (H25) at 1 °C | 1.69 | 1.25 | 0.87 | 0.61 | 0.57 | 0.55 |
| Actual haze (H90) At 20°C | 0.82 | 0.87 | 1.32 | 0.96 | 0.65 | 0.51 |
| Actual Haze (H90) at 1°C | 2.76 | 1.96 | 2.17 | 1.44 | 0.68 | 0.69 |
| Visual assessment at 8°C | Very slightly hazy | Very slightly hazy | brilliant | brilliant | brilliant | brilliant |
| Visual assessment at 1°C | Very slightly hazy | Very slightly hazy | Very slightly hazy | Very slightly hazy | brilliant | brilliant |

| Data obtained after 6 months storage at ambient temperature | | | | | | |
|---|---|---|---|---|---|---|
| Actual haze (H25) At 20 °C | 1.78 | 1.64 | 1.56 | 1.40 | 1.34 | 1.35 |
| Actual haze (H25) at 1 °C | 2.17 | 1.74 | 1.38 | 1.12 | 1.10 | 1.04 |
| Actual haze (H90) At 20°C | 1.59 | 1.49 | 2.11 | 1.52 | 1.07 | 1.04 |
| Actual Haze (H90) at 1 °C | 4.24 | 3.10 | 2.97 | 1.94 | 1.11 | 1.00 |
| Visual assessment at 8°C | Very slightly hazy | Very slightly hazy | Very slightly hazy | Very slightly hazy | brilliant | brilliant |
| Visual assessment at 1°C | Very slightly hazy | Very slightly hazy | Very slightly hazy | Very slightly hazy | brilliant | brilliant |

The data provided in Table 1 of Example 2 show that after 6 weeks of storage at ambient temperatures, upon visual assessment according to EBC 9.29 all bottled and pasteurized beers are rated 'brilliant". According to these data, use of the proline specific protease alone guarantees excellent beers even if the cold stabilization period is reduced to the period required for cooling the matured beer to minus 1 degree C, that is, essentially without a holding period at this low temperature. However, as shown in Table 4, the haze stability of the beers changes when the beers are stored for longer periods. After four months of storage at ambient, all enzyme treated beers receiving a cold-stabilization from 0 up to 5 days, develop a very faint haze. Therefore, we have to conclude that if proline specific protease is used in combination with a minimal cold stabilization period, 100 % malt beers remain visually stable for a period of at least up to 6 weeks. The Batch 5 beer, treated with the proline specific protease plus PVPP, remains "brilliant" even though it was subjected to the minimal sub-zero stabilization period. So the latter finding indicates that using a proline specific protease in combination with PVPP allows a dramatic shorthening of the cold stabilization period without any detrimental effect on the long term haze stability of the beer. The fact that we are dealing with 100% malt beers, makes this finding even more surprising.

### Example 6

### Combining enzyme treatment with short stabilisation periods at elevated temperatures

Apart from the length of the cold stabilization period, also the energy required to cool beer to below 0 degrees C, adds very significant cost to the beer production process. Therefore, the benefits of using of a proline specific protease should ideally not be limited to much shorter cold stabilization periods, but should make the sub-zero stabilization temperatures redundant. To test the latter option, we initiated a new set of pilot scale brewing experiments. Six brewing trials were carried out at 20 hl scale, essentially under conditions described in the Materials & Methods section and in Example 2 of the present application. Again the effects of using a proline specific protease were compared with using PVPP and silica. Various cold stabilization protocols were used ranging from a few hours to 4 days at 0 and 7 degrees C. Because the proline specific protease plus PVPP seems to present a potent beer stabilizing combination, the conditions of Batch 5 of the experiment described in Example 2 were repeated. However, in the present experiment, the beer was not cooled to minus 1 degree C but to 7 degree C only. The latter temperature would provide a convenient option as it represents a maximum temperature typically used for bottle or keg filling.

In the first 20 hl fermentation, the matured beer was subjected to a cold stabilization period involving 1 day cooling to 0 degrees C followed by a 4 day storage period at 0 degrees C. Then the beer was filtered with kieselguhr and divided into 2 parts during the beer filtration: the first 7hl were treated by PVPP 30g/hl mixed with kieselguhr (body feed) and the last 7 hl were treated with Siligel S (Spindal, Armainvilliers, France; 40g/hl) also mixed with kieselguhr (body feed). After filtration, the beers were bottled and pasteurized. In Table 5, these two products are referred to as Batch 1 (with PVPP) and Batch 2 (with silicagel).

The second 20 hl fermentation was carried out in exactly the same way as the first fermentation. However, in this case the beer was subjected to a cold stabilization period that involved 1 day cooling to 7 degrees C followed by a 4 day storage period at 7 degrees C. These two products are referred to as Batch 3 (with PVPP) and Batch 4 (with silicagel).

Both the third and the fourth 20 hl fermentation were carried out by adding the A. niger derived proline specific endoprotease in the cold wort before pitching at a concentration of 0.125 PPU/I. The beer resulting from this third fermentation was subjected to a cold stabilisation period involving 1 day cooling to 0 degrees C followed by a 4 day storage period at 0 degrees C followed by kieselguhr filtration (without addition of either PVPP or silicagel), bottling and pasteurisation (Batch 5). The beer from the fourth fermentation was subjected to a cold stabilisation period involving 1 day cooling to 7 degrees C followed by a 4 day storage period at 7 degrees C. Again followed by kieselguhr filtration (without addition of either PVPP or silicagel), bottling and pasteurisation (Batch 6).

In the fifth fermentation, the cold stabilisation was carried out by just cooling down the matured beer to 0 degrees C (taking less than a day), after which the beer was filtered with kieselguhr. Then the resulting beer was again divided into 2 parts during the beer filtration: the first 7hl were treated with PVPP 30g/hl mixed with kieselguhr (Batch 7). The last 7 hl of this beer were treated with Siligel S 40g/hl, also mixed with kieselguhr (body feed), bottled and pasteurized (Batch 8).

In the sixth fermentation, again the A. niger derived proline specific endoprotease was added in the cold wort, before pitching at a concentration of (0.125 PPU/I). After maturation, the beer was cooled directly to 7 degrees C and treated with PVPP 30g/hl mixed with kieselguhr. After filtration, also this beer was directly bottled and pasteurized. In Table 5 the resulting beer is referred to as Batch 9.

Shortly after bottling, all beers were subjected to a large number of standard beer analyses such as alcohol, bitterness, diacetyl, polyphenol and nonenal levels. Furthermore head retention values were determined (NIBEM and Ross & Clark). The data obtained showed that the qualities of the various beers obtained are similar and no unexpected differences could be shown. Also sensory analyses showed minor differences only. As expected, the largest differences were found in the performance of the beers in various forcing tests. For example, forcing data according to EBC 9.30 and final haze after a 6 day, 60 degrees C accelerated aging test, were determined shortly after bottling of the various beers. The results of these measurements are shown in Table 5 (see "Forcing" as well as "6 days 60 °C). Table 5 also provides the various H90 turbidity data and visual assessments of the bottled beer as recorded after a storage period of 8 weeks at ambient temperature. Measurement of these H90 data was carried out as described Example 2.

**Table 5**

| | Batch 1 | Batch 2 | Batch 3 | Batch 4 | Batch 5 | Batch 6 | Batch 7 | Batch 8 | Batch 9 |
|---|---|---|---|---|---|---|---|---|---|
| Days at 0 °C | 4 | 4 | - | - | 4 | - | Cool only 0 | Cool only 0 | - |
| Days at 7 °C | - | - | 4 | 4 | - | 4 | - | - | Cool only 0 |
| + Prol enzyme | No | no | no | no | yes | Yes | no | no | yes |
| + PVPP | 30 g/hl | no | 30 g/hl | no | no | No | 30 g/hl | no | 30 g/hl |
| + Silica | No | 40 g/hl | no | 40 g/hl | no | No | no | 40 g/hl | no |
| Forcing test: EBC.9.30 Final Haze at 1°C (H90) | 1.70 | 5.07 | 3.57 | 11.4 | 1.62 | 1.58 | 1.85 | 6.30 | 0.68 |
| Forcing test: 6 days 60 °C 24 h at 1°C (H90) | 7.41 | 14.15 | 11.48 | 38.82 | 3.22 | 4.02 | 7.60 | 15.18 | 2.09 |

| Data obtained after 8 weeks storage at ambient temperature | | | | | | | | | |
|---|---|---|---|---|---|---|---|---|---|
| (H90) at 1°C | 0.62 | 2.97 | 1.13 | 6.71 | 0.97 | 1.74 | 1.14 | 3.31 | 0.63 |
| (H90) at 20 °C | 0.69 | 0.71 | 0.50 | 0.58 | 0.82 | 1.50 | 1.08 | 0.60 | 0.54 |
| Visual at 8°C | Brill. | Brill. | Brill. | Vsh | Brill. | Brill. | Vsh | Vsh | Brill. |
| Visual at 1°C | Brill. | Sh | Vsh | Hazy | Brill. | Brill. | Vsh | Sh | Brill. |

| | | | | | | | | | |
|---|---|---|---|---|---|---|---|---|---|
| "Brill".: Brilliant ,"Vsh": Very slightly hazy, "Sh": Slightly hazy. All upon visual assessment according to EBC 9.29 after storage for 24 hours. "Cool only 0" refers to a cooling to the temperature indicated without a subsequent holding period at that temperature. | | | | | | | | | |

The data provided in Table 5 demonstrate once more that use of the proline specific protease alone allows dramatically shortened cold stabilization periods (see Batch 5). Additionally the data in Table 5 demonstrate that such a short stabilization period is also feasible at elevated temperatures (see Batch 6). As such, this finding is economically highly relevant for a specific category of brewers. The data presented in Example 5 in combination with the relatively high EBC.9.30 forcing values for the beers stabilized without using the proline specific protease as shown in Table 5 of the present Example, also suggest that these stabilization conditions may not be adequate to guarantee visually brilliant beers after prolonged storage periods. If long term shelf periods are foreseen, the data presented here (Batch 9) suggest that a "cool to bottling temperature only" protocol, that is a fast cooling to around 7 degrees C, is adequate provided that a proline specific protease is combined with a PVPP treatment. Thus, the most surprising finding of the present Example is that combining the proline specific protease (optionally in combination with PVPP if long-term shelf periods are anticipated) seems to allow a complete omission of the cold stabilization period. The implication is a very significant processing shortcut: from maturation directly to bottle filling and yet generate shelf stable, visually brilliant beers.

## Claims

1. Use of a proline-specific protease for acceleration of a beer brewing process.

2. Use according to claim 1, wherein the acceleration occurs by shortening the lagering period, wherein the lagering period comprises a maturation and a stabilization phase.

3. Use according to claim 1 or 2, wherein the lagering period is restricted to less than 8 days.

4. Use according to any one of the claims 1 to 3, wherein the acceleration of the beer brewing process is achieved by reducing the duration of the stabilisation phase to less than 7 days.

5. Use according to any one of the claims 2 to 4, whereby the stabilisation phase takes place at a temperature of 2 °C to 8°C.

6. Use acccording to any one of the preceding claims in combination with an alpha acetolactate decarboxylase.

7. Use according to any one of the preceding claims in combination with polyvinylpolypyrrolidone (PVPP).

8. Use according to any one of the preceding claims in combination with a crossflow membrane filter.
